# EUROPÄISCHE PATENTSCHRIFT

(11) **EP 1 539 018 B1**
(45) Veröffentlichungstag und Bekanntmachung des Hinweises auf die Patenterteilung: **29.08.2018**
(21) Anmeldenummer: 03797290.8
(22) Anmeldetag: 10.09.2003
(51) Int. Cl.: A61C 3/02, B23B 51/02, B23C 5/10, A61B 17/16

(54) **KERAMISCHES INSTRUMENT**
CERAMIC INSTRUMENT
INSTRUMENT EN CERAMIQUE

(30) Priorität: 17.09.2002 DE 10243104
(43) Veröffentlichungstag der Anmeldung: 15.06.2005
(73) Patentinhaber: Gebr. Brasseler GmbH & Co. KG, D-32657 Lemgo (DE)
(72) Erfinder: DANGER, Karl-Heinz, 32758 Detmold (DE); KRUMSIEK, Michael, 32657 Lemgo (DE)
(74) Vertreter: Hoefer & Partner Patentanwälte mbB
(86) Internationale Anmeldenummer: PCT/EP2003/010051
(87) Internationale Veröffentlichungsnummer: WO 2004/026165

(56) Entgegenhaltungen:
- EP-A- 0 379 201
- EP-A- 0 503 822
- EP-A- 0 627 498
- US-A- 5 641 251
- US-A1- 2002 028 422
- PATENT ABSTRACTS OF JAPAN vol. 014, no. 454 (M-1031), 28. September 1990 (1990-09-28) -& JP 02 180517 A (HITACHI TOOL ENG LTD), 13. Juli 1990 (1990-07-13)
- PATENT ABSTRACTS OF JAPAN vol. 018, no. 598 (M-1704), 15. November 1994 (1994-11-15) -& JP 06 226522 A (ASAHI KOGU SEISAKUSHO:KK), 16. August 1994 (1994-08-16)

## Beschreibung

Die Erfindung bezieht sich auf ein rotierendes Instrument gemäß dem Oberbegriff des Hauptanspruchs.

Im Einzelnen bezieht sich die Erfindung auf ein rotierendes Instrument mit einem Schaft und einem an dem Schaft befestigten oder lösbar befestigbaren Arbeitsteil.

Der Stand der Technik zeigt rotierende Instrumente, beispielsweise Dentalinstrumente, Bohrer, chirurgische Sägeblätter oder Ähnliches, welche aus metallischen Werkstoffen gefertigt sind. Abhängig von dem jeweiligen Anwendungsgebiet sowie der Ausgestaltung des rotierenden Instrumentes kann es sich als nachteilig erweisen, dass metallische Abriebrückstände im Microbereich auftreten, die bei der Versorgung oder bei dem späteren Heilungsprozess nachteilig sind.

Die bekannten Instrumente werden beispielsweise auf dem Dentalgebiet bei der Erzeugung von Knochenkavitäten, bei der Bearbeitung von Knochen oder ganz allgemein beim Einsetzen von Implantaten verwendet.

Die JP 05309102 A zeigt ein Dentalinstrument mit einem metallischen Schaft und einem keramischen Arbeitsteil. Der keramische Arbeitsteil ist dabei mit Diamantpartikeln belegt, welche die zur Bearbeitung benötigten Schneiden bilden.

Die JP 02 180 517 A beschreibt einen keramischen Bohrer mit einer Rautiefe von 0,3µm.

Die EP 0 627 498 A1 zeigt ein keramisches Bauteil mit einer Rauheit zwischen 2 und 20µm.

Ein weiteres keramisches Bauelement ist aus der EP 0 503 822 A2 bekannt. Dort ist eine Rauheit zwischen 1 und 30µm.

Die in den letzten beiden Dokumenten erläuterten keramischen Bauteile weisen die angegebenen Rauheitswerte jedoch nicht im Endzustand auf, da sie jeweils noch beschichtet werden.

Der Erfindung liegt die Aufgabe zugrunde, ein rotierendes Instrument der eingangs genannten Art zu schaffen, welches bei einfachem Aufbau und einfacher, kostengünstiger Herstellbarkeit die Nachteile des Standes der Technik vermeidet und insbesondere ohne störende Einflüsse der metallischen Werkstoffe ist.

Erfindungsgemäß wird die Aufgabe durch die Merkmale des Hauptanspruchs gelöst, die Unteransprüche zeigen weitere vorteilhafte Ausgestaltungen der Erfindung.

Erfindungsgemäß ist somit vorgesehen, dass zumindest ein Teil des Arbeitsteils des Instruments aus einem keramischen Werkstoff gefertigt ist und eine Rautiefe von 0,5 bis 6µm aufweist. Bevorzugter Weise kann sich die Rautiefe auch in einem Bereich von 1 bis 2µm bewegen.

Das erfindungsgemäße rotierende Instrument zeichnet sich durch eine Reihe erheblicher Vorteile aus. Durch die Fertigung des Arbeitsteils oder zumindest eines Teils von diesem aus einem keramischen Werkstoff ergeben sich keinerlei Probleme hinsichtlich metallischen Abriebes oder metallischer Rückstände. Weiterhin kann eine Beeinflussung des Patienten durch direkten metallischen Kontakt ausgeschlossen werden.

Ein weiterer Vorteil ergibt sich beispielsweise auch bei Keramikverblendungen auf dem Dentalgebiet. Durch die Vermeidung metallischen Abriebes entfällt auch jedwede optische Beeinträchtigung, so wie sie beispielsweise bei metallischen Werkzeugen oder Instrumenten als dunkle Färbung auftritt. Hierdurch ergibt sich eine weitaus bessere optische Gesamtwirkung, welche die nachfolgende Bearbeitung oder nachfolgende Arbeitsgänge entscheidend verbessert.

Durch die erfindungsgemäß vorgesehene Rauhtiefe ergibt sich bei den sehr kleinen Abmessungen von Dentalinstrumenten oder chirurgischen Instrumenten eine beachtliche Erhöhung der Festigkeit. So wird insbesondere das Auftreten von Mikrorissen, welches zu einem Bruch und damit zu einem Versagen des Instruments führen würde, in zuverlässiger Weise vermieden. Die erfindungsgemäßen rotierenden Instrumente zeichnen sich trotz ihrer geringen Abmessungen und ihrer geringen Durchmesser durch ein Höchstmaß an Festigkeit aus. Eine weitere Maßnahme zur Erhöhung der Stabilität und zur Verminderung von Kerbwirkungen besteht erfindungsgemäß darin, dass alle geometrisch bedingten Formübergänge des keramischen Teils des Arbeitsteils mindestens Radien von 0,5 mm aufweisen. Hierdurch werden sämtlichen scharfkantigen Übergänge, beispielsweise bei Durchmesseränderungen, bei dem Einbringen von Spannuten oder ähnlichem, vermieden. Die angegebenen Radien können sich auch auf den Rohling (Grünling) der erfindungsgemäßen Instrumente beziehen, aus dem dann das fertige Instrument gesintert oder gebrannt wird.

Eine weitere Maßnahme zur Erhöhung der Stabilität der erfindungsgemäßen Instrumente, welche mit sehr hohen Drehzahlen betrieben werden, so wie dies beispielsweise bei Dentalbohrern oder ähnlichem üblich ist, besteht darin, eine Kernverstärkung vorzusehen. Diese wird durch eine Verringerung der Tiefe von Nuten oder Einschliffen vom freien Ende des Arbeitsteils zum gegenüberliegenden Bereich des Arbeitsteils, welche an den Schaft angrenzt, hervorgerufen. Diese Kernverstärkung, welche somit eine imaginäre kegelige Grundform bildet, kann beispielsweise mit einem Winkel von 0,25 bis 3° zum Schaft hin zunehmen. Ein bevorzugter Wert liegt im Bereich von 1°.

Um das Auftreten von Mikrorissen oder ähnlichem zu vermeiden, ist bevorzugter Weise eine Mikroverdichtung des Gefüges der Oberfläche des keramischen Teils des Arbeitsteils vorgesehen. Hierdurch lässt sich die Biegefestigkeit der erfindungsgemäßen Instrumente erheblich steigern. Die Mikroverdichtung kann durch Strahlen der Oberfläche erfolgen.

Zum Schutz gegen Abrieb oder zur Verbesserung der Reibeigenschaften kann es vorteilhaft sein, die Oberfläche des keramischen Teils des Arbeitsteils mit einer Hartschicht zu versehen. Diese kann beispielsweise eine TiN-Schicht oder eine DLC-Schicht sein.

Der Einsatz der erfindungsgemäßen Keramik-Instrumente wird durch eine Tiefenmarkierung verbessert. Diese kann durch eingeschliffene Nuten, welche im Nutgrund (wie oben erwähnt) abgerundet sind oder durch eine Laserung erfolgen. Beim Markieren mittels Laser ist eine komplette Schwärzung des zu markierenden Bereichs der Keramikoberfläche möglich. Alternativ hierzu ist es auch möglich, eine Struktur oder geometrische Form aufzubringen, welche nicht umlaufend ausgeführt sein muss und erst während der Rotation des Instruments den Eindruck einer kontinuierlichen Schwärzung hinterlässt.

Die Tiefenmarkierung kann beispielsweise eine Rauhtiefe im Bereich von 1 bis 10 µm aufweisen, ein bevorzugter Bereich ist 2 bis 4 µm.

Erfindungsgemäß ist es möglich, den Arbeitsteil und den Schaft aus dem keramischen Werkstoff zu fertigen. Es ist sowohl möglich, einzelne keramische Bauelemente zu fügen, als auch das rotierende Instrument einstückig herzustellen.

Eine weitere, vorteilhafte Ausgestaltung der Erfindung liegt darin, dass der Arbeitsteil einen metallischen Träger und zumindest eine auf diesen aufgebrachte Lage aus dem keramischen Werkstück umfasst. Anstelle der Lage ist es auch möglich, ein zusätzliches keramisches Bauelement aufzubringen. Die Verbindung kann dabei beispielsweise mittels eines Klebers erfolgen, beispielsweise mittels eines temperaturstabilen Komposits.

Um die Oberfläche des keramischen Werkstoffes porenfrei und glatt auszubilden, kann es günstig sein, wenn die Oberfläche geschliffen oder poliert wird.

Erfindungsgemäß ist es möglich, den keramischen Werkstoff mit Schneiden und/oder einer Verzahnung zu versehen. So ist es beispielsweise möglich, das erfindungsgemäße Instrument als Sägeblatt auszubilden, um Knochenschnitte oder Ähnliches durchzuführen.

Insgesamt kann das erfindungsgemäße rotierende Instrument als Dentalinstrument, beispielsweise als Knochenbohrer oder Ähnliches, ausgebildet sein. Es ist jedoch auch möglich, dieses als reines Schleif- oder Trennwerkzeug auszugestalten, beispielsweise auch als Sägeblatt.

Als besonders vorteilhaft hat es sich erwiesen, wenn als keramischer Werkstoff ein Aluminiumoxid und/oder ein Zirkonoxid verwendet wird. Vorteilhaft können auch Mischungen dieser beiden Oxide sein (beispielsweise Al₂O₃ oder ZrO₂). Eine derartige Mischkeramik weist hinsichtlich der biegefestigkeit und der Zähigkeit hervorragende Eigenschaften auf. Eine weitere Variante besteht darin, Zirkonoxid mit tetragonalen Zirkon-Polykrystallen zu verwenden. Eine derartige Keramik weist ein noch feineres Korn und damit verbesserte mechanische Eigenschaften, insbesondere eine erhöhte Bruchfestigkeit auf. Durch eine entsprechende Al₂O₃-Dotierung ergibt sich eine verbesserte Alterungsbeständigkeit. Ein derartiger Werkstoff weist eine hervorragende Biokompatibilität auf.

Die erfindungsgemäßen Instrumente können auch mit einer Innenkühlung versehen sein. Hierzu dient beispielsweise eine durchgehende Bohrung, welche bevorzugter Weise mehrere Austrittsöffnungen im Bereich des Arbeitsteils des Instruments hat.

Das erfindungsgemäße Instrument kann beispielsweise mit zwei bis drei Schneiden versehen sein, es ist jedoch auch möglich, nur eine Schneide vorzusehen oder eine größere Anzahl von Schneiden, beispielsweise vier bis zehn Schneiden.

Auch die Verzahnung oder der Schliff der Schneiden kann variiert werden. So ist es möglich, eine doppelte Verzahnung oder Kreuzverzahnung vorzusehen oder insgesamt drei Verzahnungen oder Schliffe aufzubringen.

Das erfindungsgemäße Instrument kann als Bohr- oder Fräsinstrument ausgebildet sein, der Arbeitsteil kann kugelförmig, zylinderisch, zylindrisch-rund, konisch, konischrund, abgestuft oder als Gewindeschneider ausgeführt sein.

Im Folgenden wird die Erfindung anhand von Ausführungsbeispielen in Verbindung mit der Zeichnung beschrieben. Dabei zeigt:
- Fig. 1: eine Seitenansicht, teils im Schnitt, eines ersten Ausführungsbeispiels eines erfindungsgemäßen Instruments in Form eines Dentalbohrers,
- Fig. 2: eine Seitenansicht eines weiteren Ausgestaltungsbeispiels eines erfindungsgemäßen Dentalinstruments, welches in Form eines Kronentrenners ausgebildet ist,
- Fig. 3: eine Seitenansicht eines weiteren Ausgestaltungsbeispiels eines erfindungsgemäßen Instruments, welches als Implantologievorbohrer ausgestaltet ist,
- Fig. 4: eine vereinfachte Seitenansicht eines weiteren Ausführungsbeispiels eines erfindungsgemäßen Instruments in Form eines Knochenfräsers,
- Fig. 5: eine Seitenansicht des in Fig. 4 gezeigten Instruments in einer um 90° gedrehten Darstellung,
- Fig. 6: eine Schnittansicht längs der Linie A-A von Fig. 5,
- Fig. 7: eine Seitenansicht eines als Implantologie-Vorbohrer ausgebildeten Instruments,
- Fig. 8: eine stirnseitige Ansicht des in Fig. 7 gezeigten Instruments, und
- Fig. 9: Detail-Darstellungen der Einzelheiten Z von Fig. 7.

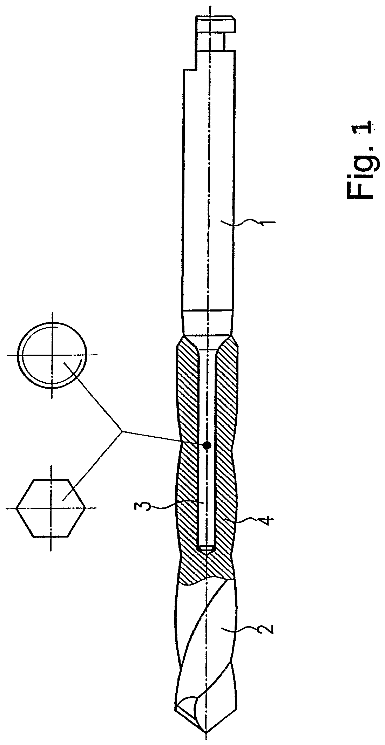

Das in Fig. 1 gezeigte erfindungsgemäße rotierende Instrument ist in Form eines Bohrers ausgebildet. Dieser umfasst einen Schaft 1 sowie ein Arbeitsteil 2, welches, wie bei einem Bohrer üblich, mit Schneiden versehen ist. Auf die Darstellung und Beschreibung des Arbeitsteils 2 kann im Detail verzichtet werden, da derartige Bohrer aus dem Stand der Technik vorbekannt sind.

Erfindungsgemäß ist an dem Schaft 1 ein bolzen- oder dornförmiger Träger 3 befestigt bzw. einstückig mit dem Schaft 1 verbunden. Der Träger 3 ist in einer zentrischen Ausnehmung des Arbeitsteils 2 angeordnet und somit von einer Lage 4 aus einem keramischen Werkstoff umgeben.

Wie die Detail-Ansichten der Fig. 1 zeigen, kann der Träger 3 beispielsweise einen sechseckigen Querschnitt aufweisen. Er kann auch mit einem runden Querschnitt versehen sein, der beispielsweise mit Nuten oder einem Gewinde ausgestattet ist.

Der Schaft 1 sowie der Träger 3 können aus Stahl gefertigt sein, beispielsweise aus RF-Stahl.

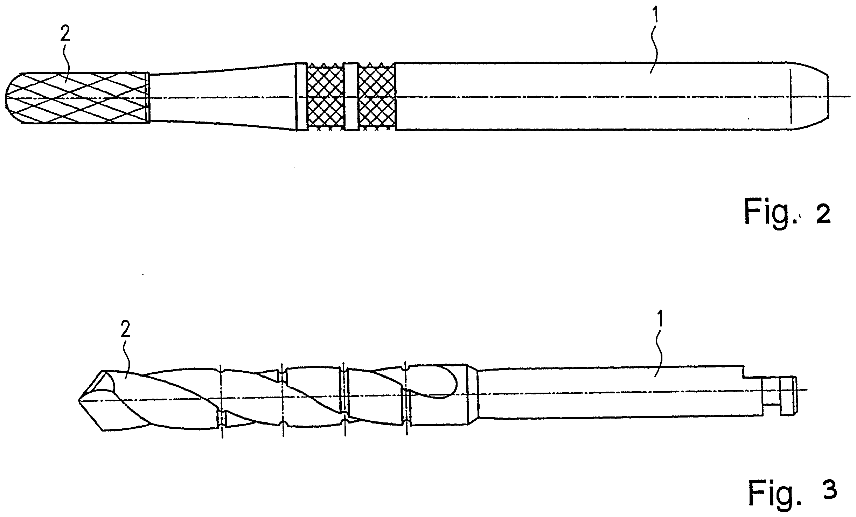

Die Fig. 2 und 3 zeigen weitere Ausgestaltungsformen erfindungsgemäßer rotierender Instrumente, bei welchen der Arbeitsteil 2 jeweils aus einem keramischen Werkstoff besteht bzw. mit einer Lage aus einem keramischen Werkstoff versehen ist. Das in Fig. 2 gezeigte Instrument ist in Form eines Kronentrenners ausgebildet, während das in Fig. 3 gezeigte Instrument einen Implantologievorbohrer zeigt.

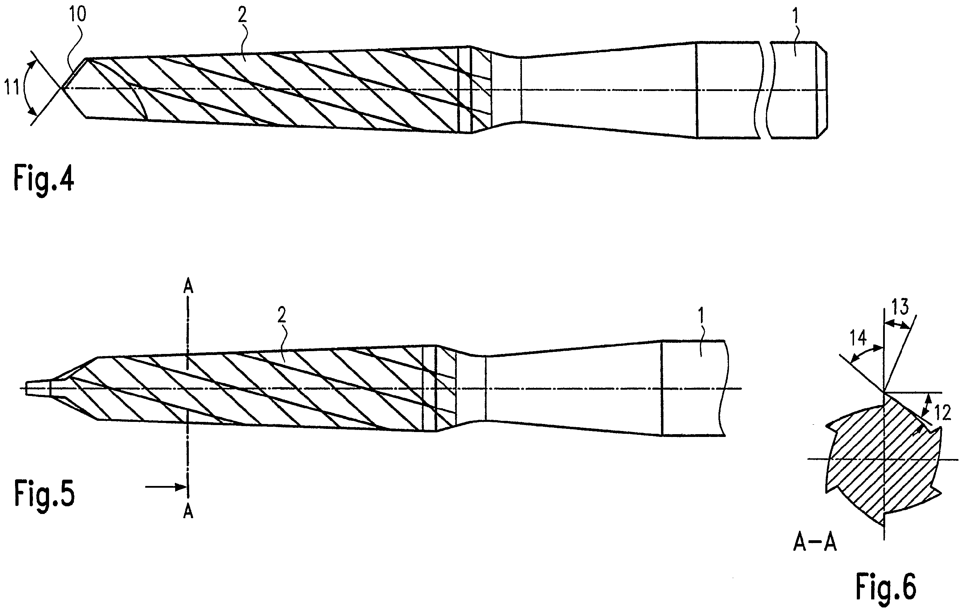

Die Fig. 4 bis 6 zeigen ein Ausführungsbeispiel, bei welchem das erfindungsgemäße Instrument in Form eines Knochenfräsers ausgebildet ist. Der Arbeitsteil 2 kann einstückig mit dem Schaft ausgebildet sein. Weiterhin weist das Arbeitsteil 2 sich kreuzende Schliffe oder Verzahnungen auf, so wie dies in den Fig. 4 und 5 dargestellt ist. Die Spitze des in den Fig. 4 und 5 gezeigten Knochenfräsers weist einen Freischliff 10 auf, der beispielsweise 10° betragen kann. Mit dem Bezugszeichen 11 ist ein Spitzenwinkel angegeben, der beispielsweise 100° betragen kann. Der Durchmesser im Bereich der Spitze kann beispielsweise 1,44 mm betragen, während die Länge des Arbeitsteils 2 10 mm beträgt. Die Gesamtlänge des Instruments beträgt beispielsweise 44,5 mm.

Die weiteren Bemaßungen des Instruments können wie folgt sein:

| | |
|---|---|
| Arbeitsteillänge: | 1-25 mm |
| Arbeitsteildurchmesser: | 1-25 mm |
| Spitzenfreischliff: | 0-40° |
| Spitzenwinkel: | 50-150° |
| Freiwinkel: | 0°-70° |
| Spanwinkel: | -10°-20° |
| Drall: | 0-80° |
| Zähnezahl: | 1-20 |

Es ist auch eine Kreuzverzahnung mit einem Drall von 20° bis 70° möglich. Auch ein Querhieb zur Optimierung des Schneidverhaltens mit einer Steigung von 0,5 bis 2,0 mm ist in bevorzugter Weiterentwicklung günstig.

In Fig. 6 sind die möglichen Winkel nochmals dargestellt. Dabei ist beispielsweise ein Freiwinkel 12 von 35° gezeigt. Ein Spanwinkel 13 kann beispielsweise 0° betragen (zur Verdeutlichung der Darstellung ist der Spanwinkel 13 überzeichnet). Ein Keilwinkel 14 beträgt beispielsweise 50°.

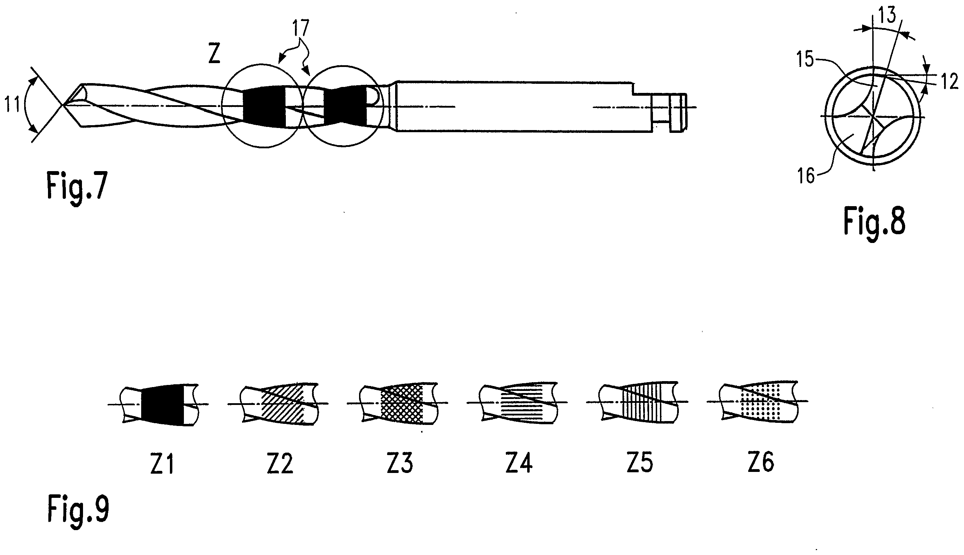

Die Fig. 7 bis 9 zeigen ein weiteres Ausführungsbeispiel, bei welchem das Instrument in Form eines Implantologie-Vorbohrers (Pilotbohrer) ausgebildet ist. Dieser kann beispielsweise folgende Abmessungen haben:

| | |
|---|---|
| Arbeitsteillänge: | 1-25 mm |
| Arbeitsteildurchmesser: | 1-8 mm |
| Spitzenfreischliff: | 0-40° |
| Spitzenwinkel: | 50-150° |
| Freiwinkel: | 0-60° |
| Spanwinkel: | 0-20° |
| Drall: | 0-60° |
| Zähnezahl: | 1-10 |

Die Fig. 8 zeigt beispielsweise einen Spanwinkel 13 von 10° sowie einen Freiwinkel 12 von 8°. Mit dem Bezugszeichen 15 ist ein Freischliff der Schneide angegeben, der beispielsweise 12° beträgt, während das Bezugszeichen 16 einen Freischliff der Schneide von 25° zeigt.

Der in den Fig. 7 bis 9 gezeigte Implantologie-Vorbohrer kann mit einer oder mehreren Tiefenmarkierungen 17 versehen sein. Diese können, wie in Fig. 9 dargestellt, unterschiedliche optische Formen haben. Die Einzelheit Z1 zeigt eine gänzliche Schwärzung, die Einzelheit Z2 zeigt eine schräggestellte Linienmarkierung, in der Einzelheit Z3 ist eine Kreuzmarkierung vorgesehen, während die Einzelheiten Z4 und Z5 jeweils parallele Linien umfassen. Die Einzelheit Z6 zeigt eine Ausführungsvariante mit einem Punktmuster.

Die erfindungsgemäß verwendeten, oben beschriebenen Keramiken können folgende Werte aufweisen:

| | |
|---|---|
| Härte: | 1200-1500 |
| Dichte: | 5,4-6,1 |
| Biegefestigkeit: | 1200-3000 |

Die Erfindung ist nicht auf die gezeigten Ausführungsbeispiele beschränkt. Vielmehr ergeben sich im Rahmen der Erfindung vielfältige Abwandlungs- und Modifikationsmöglichkeiten.

### Bezugszeichenliste

- 1: Schaft
- 2: Arbeitsteil
- 3: Träger
- 4: Lage
- 10: Freischliff
- 11: Spitzenwinkel
- 12: Freiwinkel
- 13: Spanwinkel
- 14: Keilwinkel
- 15: Freischliff
- 16: Freischliff
- 17: Tiefenmarkierung

## Patentansprüche

1. Rotierendes Instrument mit einem Schaft (1) und einem an dem Schaft befestigten oder lösbar befestigbaren Arbeitsteil (2), wobei zumindest ein Teil des Arbeitsteils (2) aus einem keramischen Werkstoff gefertigt ist, und wobei das keramische Teil des Arbeitsteils (2) eine Rauhtiefe von 0,5 bis 6 µm aufweist, **dadurch gekennzeichnet, dass** alle geometrisch bedingten Formübergänge des keramischen Teils des Arbeitsteils (2) mindestens Radien von 0,5 mm aufweisen, und dass der Arbeitsteil (2) mit einer durch Verringerung der Tiefe von Nuten oder Einschliffen vom freien Ende zum Schaft des Arbeitsteils (2) hervorgerufenen Kernverstärkung versehen ist.

2. Instrument nach Anspruch 1, **dadurch gekennzeichnet, dass** der keramische Teil des Arbeitsteils (2) eine Rauhtiefe von 1 bis 2 µm aufweist.

3. Instrument nach Anspruch 1, **dadurch gekennzeichnet, dass** die Kernverstärkung eine im Wesentlichen kegelige Grundform hat.

4. Instrument nach Anspruch 1 oder 3, **dadurch gekennzeichnet, dass** der Kerndurchmesser von 0,25 bis 3° zum Schaft hin zunimmt.

5. Instrument nach einem der Ansprüche 1 bis 4, **dadurch gekennzeichnet, dass** der Kerndurchmesser um 1° zum Schaft hin zunimmt.

6. Instrument nach einem der Ansprüche 1 bis 5, **dadurch gekennzeichnet, dass** die Oberfläche des keramischen Teils des Arbeitsteils (2) mikroverdichtet ist.

7. Instrument nach einem der Ansprüche 1 bis 6, **dadurch gekennzeichnet, dass** die Oberfläche des keramischen Teils des Arbeitsteils (2) durch Strahlen der Oberfläche mikroverdichtet ist.

8. Instrument nach einem der Ansprüche 1 bis 7, **dadurch gekennzeichnet, dass** die Oberfläche des keramischen Teils des Arbeitsteils (2) mit einer Hartschicht versehen ist.

9. Instrument nach einem der Ansprüche 1 bis 8, **dadurch gekennzeichnet, dass** die Oberfläche des keramischen Teils des Arbeitsteils (2) zumindest eine Tiefenmarkierung aufweist.

10. Instrument nach Anspruch 9, **dadurch gekennzeichnet, dass** die Tiefenmarkierung eine Rauhtiefe von 1 bis 10 µm aufweist.

11. Instrument nach einem der Ansprüche 9 oder 10, **dadurch gekennzeichnet, dass** die Tiefenmarkierung eine Rauhtiefe von 2 bis 4 µm aufweist.

12. Instrument nach einem der Ansprüche 9 bis 11, **dadurch gekennzeichnet, dass** die Tiefenmarkierung eine Lasermarkierung ist.

13. Instrument nach einem der Ansprüche 9 bis 11, **dadurch gekennzeichnet, dass** die Tiefenmarkierung eingeschliffene Nuten umfasst.

14. Instrument nach einem der Ansprüche 1 bis 13, **dadurch gekennzeichnet, dass** der Arbeitsteil (2) und der Schaft (1) aus einem keramischen Werkstoff gefertigt sind.

15. Instrument nach einem der Ansprüche 1 bis 14, **dadurch gekennzeichnet, dass** der Arbeitsteil (2) einen metallischen Träger (3) und zumindest eine auf diesen aufgebrachte Lage (4) aus dem keramischen Werkstoff umfasst.

16. Instrument nach Anspruch 15, **dadurch gekennzeichnet, dass** die Lage (4) aus dem keramischen Werkstoff mit dem Träger (3) mittels eines Klebers verbunden ist.

17. Instrument nach einem der Ansprüche 1 bis 16, **dadurch gekennzeichnet, dass** die Oberfläche des keramischen Werkstoffs geschliffen ist.

18. Instrument nach einem der Ansprüche 1 bis 17, **dadurch gekennzeichnet, dass** der keramische Werkstoff mit Schneiden und/oder einer Verzahnung versehen ist.

19. Instrument nach einem der Ansprüche 1 bis 18, **dadurch gekennzeichnet, dass** der keramische Werkstoff Aluminiumoxid und/oder Zirkonoxid umfasst.

20. Instrument nach einem der Ansprüche 1 bis 19, **dadurch gekennzeichnet, dass** dieses als Dentalinstrument ausgebildet ist.

21. Instrument nach einem der Ansprüche 1 bis 19, **dadurch gekennzeichnet, dass** dieses als Bohrer ausgebildet ist.

22. Instrument nach einem der Ansprüche 1 bis 21, **dadurch gekennzeichnet, dass** zumindest ein Kühlmittelkanal zumindest in dem Arbeitsteil (2) des Instruments ausgebildet ist.

## Claims

1. A rotating instrument with a shaft (1) and a working part (2) attached or detachably attachable to the shaft, wherein at least one part of the working part (2) is manufactured from a ceramic material, and wherein the ceramic part of the working part (2) has a surface roughness of 0.5 to 6 µm, **characterised in that** all geometry-related shape transitions of the ceramic part of the working part (2) at least have radii of 0.5 mm, and that the working part (2) is provided with a core reinforcement induced by reducing the depth of grooves or grindings from the free end to the shaft of the working part (2).

2. The instrument according to claim 1, **characterised in that** the ceramic part of the working part (2) has a surface roughness of 1 to 2 µm.

3. The instrument according to claim 1, **characterised in that** the core reinforcement has an essentially tapered basic shape.

4. The instrument according to claim 1 or 3, **characterised in that** the core diameter increases from 0.25 to 3° towards the shaft.

5. The instrument according to any one of claims 1 to 4, **characterised in that** the core diameter increases by 1° towards the shaft.

6. The instrument according to any one of claims 1 to 5, **characterised in that** the surface of the ceramic part of the working part (2) is micro-compacted.

7. The instrument according to any one of claims 1 to 6, **characterised in that** the surface of the ceramic part of the working part (2) is micro-compacted by blasting the surface.

8. The instrument according to any one of claims 1 to 7, **characterised in that** the surface of the ceramic part of the working part (2) is provided with a hard coat.

9. The instrument according to any one of claims 1 to 8, **characterised in that** the surface of the ceramic part of the working part (2) has at least one depth mark.

10. The instrument according to claim 9, **characterised in that** the depth mark has a surface roughness of 1 to 10 µm.

11. The instrument according to any one of claims 9 or 10, **characterised in that** the depth mark has a surface roughness of 2 to 4 µm.

12. The instrument according to any one of claims 9 to 11, **characterised in that** the depth mark is a laser marking.

13. The instrument according to any one of claims 9 to 11, **characterised in that** the depth mark comprises ground-in grooves.

14. The instrument according to any one of claims 1 to 13, **characterised in that** the working part (2) and the shaft (1) are manufactured from a ceramic material.

15. The instrument according to any one of claims 1 to 14, **characterised in that** the working part (2) comprises a metal substrate (3) and at least one layer (4) of the ceramic material applied thereon.

16. The instrument according to claim 15, **characterised in that** the layer (4) of the ceramic material is bound to the substrate (3) by means of an adhesive.

17. The instrument according to any one of claims 1 to 16, **characterised in that** the surface of the ceramic material is ground.

18. The instrument according to any one of claims 1 to 17, **characterised in that** the ceramic material is provided with cutting edges and/or toothing.

19. The instrument according to any one of claims 1 to 18, **characterised in that** the ceramic material comprises aluminium oxide and/or zirconium oxide.

20. The instrument according to any one of claims 1 to 19, **characterised in that** this is formed as a dental instrument.

21. The instrument according to any one of claims 1 to 19, **characterised in that** this is formed as a drill.

22. The instrument according to any one of claims 1 to 21, **characterised in that** at least one coolant channel is formed at least in the working part (2) of the instrument.

## Revendications

1. Instrument rotatif avec une tige (1) et une partie de travail (2) fixée sur la tige ou pouvant être fixée de manière amovible, dans lequel au moins une partie de la partie de travail (2) est fabriquée en un matériau céramique, et dans lequel la partie céramique de la partie de travail (2) présente une profondeur de rugosité de 0,5 à 6 µm, **caractérisé en ce que** toutes les transitions de forme conditionnées géométriquement de la partie céramique de la partie de travail (2) présentent au moins des rayons de 0,5 mm, et que la partie de travail (2) est pourvue d'un renforcement de coeur suscité par diminution de la profondeur de rainures ou entaille de l'extrémité libre à la tige de la partie de travail (2).

2. Instrument selon la revendication 1, **caractérisé en ce que** la partie céramique de la partie de travail (2) présente une profondeur de rugosité de 1 à 2 µm.

3. Instrument selon la revendication 1, **caractérisé en ce que** le renforcement de coeur présente une forme de base sensiblement conique.

4. Instrument selon la revendication 1 ou 3, **caractérisé en ce que** le diamètre de coeur augmente de 0,25 à 3° vers la tige.

5. Instrument selon l'une quelconque des revendications 1 à 4, **caractérisé en ce que** le diamètre de coeur augmente de 1° vers la tige.

6. Instrument selon l'une quelconque des revendications 1 à 5, **caractérisé en ce que** la surface de la partie céramique de la partie de travail (2) est microcompactée.

7. Instrument selon l'une quelconque des revendications 1 à 6, **caractérisé en ce que** la surface de la partie céramique de la partie de travail (2) est microcompactée par rayonnement de la surface.

8. Instrument selon l'une quelconque des revendications 1 à 7, **caractérisé en ce que** la surface de la partie céramique de la partie de travail (2) est pourvue d'une couche dure.

9. Instrument selon l'une quelconque des revendications 1 à 8, **caractérisé en ce que** la surface de la partie céramique de la partie de travail (2) présente au moins un marquage profond.

10. Instrument selon la revendication 9, **caractérisé en ce que** le marquage profond présente une profondeur de rugosité de 1 à 10 µm.

11. Instrument selon l'une quelconque des revendications 9 ou 10, **caractérisé en ce que** le marquage profond présente une profondeur de rugosité de 2 à 4 µm.

12. Instrument selon l'une quelconque des revendications 9 à 11, **caractérisé en ce que** le marquage profond est un marquage laser.

13. Instrument selon l'une quelconque des revendications 9 à 11, **caractérisé en ce que** le marquage profond comporte des rainures entaillées.

14. Instrument selon l'une quelconque des revendications 1 à 13, **caractérisé en ce que** la partie de travail (2) et la tige (1) sont fabriquées en un matériau céramique.

15. Instrument selon l'une quelconque des revendications 1 à 14, **caractérisé en ce que** la partie de travail (2) comporte un support métallique (3) et au moins une couche (4) appliquée sur celui-ci en matériau céramique.

16. Instrument selon la revendication 15, **caractérisé en ce que** la couche (4) en matériau céramique est raccordée au support (3) au moyen d'une colle.

17. Instrument selon l'une quelconque des revendications 1 à 16, **caractérisé en ce que** la surface du matériau céramique est poncée.

18. Instrument selon l'une quelconque des revendications 1 à 17, **caractérisé en ce que** le matériau céramique est pourvu de tranchants et/ou d'une denture.

19. Instrument selon l'une quelconque des revendications 1 à 18, **caractérisé en ce que** le matériau céramique comporte de l'oxyde d'aluminium et/ou de l'oxyde de zirconium.

20. Instrument selon l'une quelconque des revendications 1 à 19, **caractérisé en ce que** celui-ci est réalisé en tant qu'instrument dentaire.

21. Instrument selon l'une quelconque des revendications 1 à 19, **caractérisé en ce que** celui-ci est réalisé en tant que foret.

22. Instrument selon l'une quelconque des revendications 1 à 21, **caractérisé en ce qu'**au moins un canal de réfrigérant est réalisé au moins dans la partie de travail (2) de l'instrument.
